# EUROPEAN PATENT SPECIFICATION

(11) **EP 0 636 377 B1**
(45) Date of publication and mention of the grant of the patent: **20.02.2002**
(21) Application number: 94305536.8
(22) Date of filing: 27.07.1994
(51) Int. Cl.: A61L 31/00, A61L 27/00

(54) **Use of a composite surgical material**
Anwendung eines chirurgischen Verbundmaterials
Utilisation d'un matériau composite chirurgical

(30) Priority: 28.07.1993 GB 9315614; 17.09.1993 GB 9319273
(43) Date of publication of application: 01.02.1995
(73) Proprietor: JOHNSON & JOHNSON MEDICAL, INC., Arlington, Texas 76014 (US)
(72) Inventor: Harvey, Wilson, Gargunnock, Stirling, FK8 3AX (GB); Light, Nicholas, Doune, Perthshire FK16 6DE (GB); Haynes, Carla, Cambuslang, Glasgow G72 8NH (GB)
(74) Representative: Fisher, Adrian John

(56) References cited:
- EP-A- 0 194 192
- EP-A- 0 297 535
- EP-A- 0 567 234
- WO-A-90/13302
- US-A- 5 037 377

## Description

The present invention relates to the use of bioabsorbable composite surgical materials.

The use of bioabsorbable materials (also called resorbable materials or absorbable materials) in surgery is by now quite well known. The materials undergo gradual degradation when they are introduced into the human or animal body. This degradation results from hydrolysis which takes place on contact with living tissue in the presence of proteolytic enzymes contained therein. The hydrolysis fragments from the hydrolysis of the bioabsorbable materials are non-toxic and readily absorbed by the human or animal body.

For example, bioabsorbable surgical sutures made from copolymers of lactic and glycolic acids are in widespread use. Such sutures do not need to be removed from the wound site after wound healing is complete. Instead, the sutures undergo slow hydrolysis and absorption by the body.

Other bioabsorbable surgical materials have been used as temporary prostheses in repair surgery. For example, sheets of bioabsorbable material may be used as prostheses for regions of the pericardium or the peritoneal membrane. Healing of the damaged membrane takes place from the edges of the prosthesis, and the prosthesis is gradually absorbed as healing progresses.

Likewise, tubes of bioabsorbable surgical material have been used as arterial grafts. Once again, healing of the damaged artery is accompanied by gradual resorption of the graft.

EP-A-0194192 describes a bioabsorbable composite material that is especially suitable for use as a surgical prosthesis. The material comprises a sheet of reconstituted collagen reinforced with a mesh of a synthetic bioabsorbable polymer such as polylactic/polyglycolic acid or oxidised regenerated cellulose. The composite material is sufficiently strong to be used as an arterial graft or the like, and in particular is strong enough to hold sutures. The reconstituted collagen sheet fills all of the interstices in the mesh and renders the composite material leak-proof. The leak-proofing effect is especially important when the material is to be used to repair blood vessels. Furthermore, it has been found that the reinforced collagen films are absorbed more slowly *in vivo* than non-reinforced collagen films.

A defect of the reinforced collagen films described in EP-A-0194192 is that the film does not remain leak-proof in use for a sufficiently long time to be usable for some surgical applications. This is to say, the rate of bioabsorption of the collagen is sufficiently rapid that in some cases the film may start to leak through the interstices of the reinforcing mesh before wound healing is complete.

The rate of absorption of collagen *in vivo* can be reduced by chemical cross-linking of the collagen with succinimide or glutaraldehyde. However, such chemical treatment necessarily renders the collagen less biocompatible.

It has now been found that reinforced collagen films similar to those described in EP-A-0194192 may be made with significantly improved leak-proofing characteristics by incorporating oil droplets into the collagen layer.

The present invention provides use of a composite material comprising a collagen matrix reinforced by a layer of a synthetic bioabsorbable material and having oil droplets dispersed in the collagen matrix for the preparation of a bioabsorbable membrane graft in the form of a sheet for use in surgery.

The present invention further provides use of a composite material comprising a collagen matrix reinforced by a layer of a synthetic bioabsorbable material and having oil droplets dispersed in the collagen matrix for the preparation of a bioabsorbable graft in the form of a tube for use in surgery.

Preferably, the oil droplets comprise from 2% to 75% by weight of the composite material, more preferably from 5% to 50% by weight of the composite material, and most preferably from 10% to 40% by weight of the composite material.

The oil droplets are preferably microdroplets such that at least 90% of the droplets have diameters in the range 0.1µm to 250µm. More preferably, at least 90% of the droplets have diameters in the range 1µm to 50µm.

The oil droplets are preferably distributed uniformly throughout the collagen matrix. However, in certain embodiments, the oil droplets may be distributed non-uniformly, for example to provide different rates of bioabsorption of the collagen matrix in different regions of the composite material.

The oil may be any bioabsorbable and biocompatible oil. For example, vegetable oils such as corn oil, sunflower seed oil, sesame seed oil or linseed oil may be used. The term "oil" also encompasses oleaginous materials, such as lanolin, that are solid or semisolid at room temperature.

The collagen matrix preferably comprises insoluble fibrous collagen, such as insoluble Type I and/or Type III collagen fibres. The collagen matrix may additionally comprise soluble collagen, such as gelatin or atelocollagen, or acid - soluble collagen, or even collagen fibres reconstituted from these soluble collagens. The collagen may be obtained from any animal, fish or avian source, but is preferably obtained from bovine corium.

The relative amounts of the collagen matrix and the synthetic bioabsorbable material in the composite surgical materials used in the present invention may vary widely, depending on the intended use of the materials and the desired rate of bioabsorption. The composite materials preferably contain from 10% to 95% by weight of the collagen matrix (including the oil droplets and any other substances dispersed therein). Preferably, the composite materials contain from 20% to 60% by weight of the collagen matrix.

The reinforcing layer is formed from a synthetic bioabsorbable material. Preferred synthetic bioabsorbable materials include synthetic suture materials such as polymers or copolymers of lactic and/or glycolic acids. Other preferred synthetic bioabsorbable materials include modified celluloses, such as oxidised regenerated cellulose. Particularly preferred synthetic bioabsorbable materials include the polylactic/polyglycolic acid copolymer sold under the Registered Trade Mark VICRYL and the oxidised regenerated cellulose sold under the Registered Trade Mark SURGICEL.

The layer of synthetic bioabsorbable material is preferably in the form of a knitted, woven or non-woven mesh or web. This arrangement combines flexibility with sufficient strength for the composite material to hold sutures. The foraminous nature of these reinforcing layers also assists suturing. The mesh size selected for the layer of synthetic bioabsorbable material can vary widely, depending on the particular surgical application that is envisaged.

The composite surgical materials used in the present invention preferably further comprise pharmacologically active agents dispersed in the collagen matrix. Preferred pharmacologically active agents include antibiotics, antiseptics, anti-inflammatory agents and agents that promote wound healing, such as cytokines or glycosaminoglycans (e.g. hyaluronic acid and its salts, heparin and the like). The pharmacologically active agents are preferably present in an amount of 0.01%-5% by weight, more preferably 0.01%-1% by weight based on the total weight of the composite material. It will be appreciated that the presence of the oil droplets allows oleophilic active agents to be dispersed in the collagen matrix as well as hydrophilic active agents.

The composite surgical materials provided by the present invention are in the form of a flat sheet or a tube.

Flat sheets of the material provided by the present invention may be used as membrane grafts for repair of the peritoneum or pericardium. Tubes of the material provided by the present invention may be used as grafts for the repair of blood vessels. It has been found, surprisingly, that the sheets and tubes of material provided in accordance with the present invention remain leak-proof for substantially longer than corresponding materials prepared in accordance with EP-A-0194192.

The composite surgical grafts provided by the present invention may be made by a process comprising the steps of: providing a layer of a synthetic bioabsorbable material; providing a dispersion of collagen in an oil-in-water emulsion; coating at least one face of the layer of synthetic bioabsorbable material with the said dispersion; and drying the composite material thus obtained.

Preferably, the step of providing a dispersion of collagen in an oil-in-water emulsion comprises adding the collagen and the oil to water followed by emulsifying the oil at high shear. Emulsifiers may be added to assist this process, but are not always necessary, since collagen is an effective emulsifier. Where the oil is a solid or semisolid oleaginous material at room temperature (e.g. lanolin), the emulsification step is carried out at an elevated temperature, at which the oil is liquid.

Preferably, the weight ratio of collagen to oil in the emulsion is from 10:1 to 1:10, more preferably is from 2:1 to 1:5, and most preferably it is from 1:1 to 1:3. Preferably, the concentration of collagen in the emulsion is from 0.05% w/v to 10% w/v, more preferably 0.1% w/v to 5% w/v.

Preferably, the collagen, oil and synthetic bioabsorbable polymer are as defined above for the preferred embodiments of the present invention.

Specific embodiments of the present invention will now be described further, by way of example, with reference to the accompanying Figures, in which:
Figure 1 shows a photomicrograph of a cross section through a collagen/vicryl film subcutaneous implant after 14 days; and
Figure 2 shows a photomicrograph of a cross section through a collagen/oil/vicryl film subcutaneous inplant (500% oil/collagen w/w) after 14 days.

### Example 1

Reinforced collagen films for use in the present invention are prepared as follows.

Fibrous collagen obtained from bovine corium, prewashed to remove the majority of non-collagenous components as described in US-A-4614794 or US-A-4320201 is suspended in clear, deionised pyrogen-free water and homogenised to a fine fibrous suspension by passage through a homogenising system, such as that described in US-A-4320201. The collagen suspension is then acidified by addition of acetic acid at a concentration of 0.05M. The concentration of collagen fibres in the dispersion is 0.5% w/v.

To this suspension is added sesame seed oil at 50%, 100%, 200%, or 500% (as % of the collagen content, w/w). The mixture is homogenised to form an emulsion, degassed under vacuum, and poured into trays. In the trays a mesh of poly(L-lactide) poly(L-glycolide) (supplied by Ethicon Inc. under the Registered Trade Mark VICRYL, style 9) is immersed in the collagen/oil emulsion. The emulsions are dried in air at room temperature to form films.

For comparison purposes a film was made in identical fashion, but with zero oil content.

### Example 2

The permeability to physiological saline of films prepared in accordance with Example 1 is determined as follows.

Pieces of collagen/oil/Vicryl™ film (oil content with respect to collagen: 0 (comparative example), 50%, 200%, 500%) are clamped between two hollow, flanged, cylindrical chambers (2.5cm diameter) to form a water-tight seal. This apparatus is suspended vertically over a beaker in a humidified chamber at 37°C. 25 ml of phosphate-buffered saline is placed in the upper chamber. The volume permeating through the film is collected in the beaker and measured daily, at which time the volume of saline in the upper chamber is replenished to 25ml.

The results are expressed as the cumulated volume of saline which has passed through the films. During the first 9 days there is no significant difference in permeability between the different films. However, from days 10 to 17 there is a significant variation in permeability which correlates with the oil content of the films:

| Oil content (%) | vol.(ml)days 1-9 | vol.(ml)days 10-17 |
|---|---|---|
| 0 (comparative) | 81 | 137 |
| 50 | 70 | 106 |
| 200 | 74 | 101 |
| 500 | 82 | 82 |

### Example 3

The effect of oil content on the susceptibility of reinforced collagen films to degradation by collagenase is determined as follows.

Pieces of film prepared as in Example 1 are cut to give a collagen content of approx. 50mg. These are incubated at 37°C in 32.5 ml of Tris buffer (pH7.2) containing bacterial collagenase (Clostridiopeptidase A) at 50 U/ml for 2.5h. collagen degradation is measured by hydroxyproline assay of aliquots of the supernatant solution after centrifugation, and expressed as a % of the starting collagen content of the sample.

Collagen degradation was significantly decreased in films containing oil at 200% and 500% of the weight of collagen (degradation: 63.9% and 49.2%, respectively, compared with 85.0% degradation of collagen film containing no oil).

### Example 4

The effect of oil content on the susceptibility of reinforced collagen films to degradation *in vivo* is determined as follows.

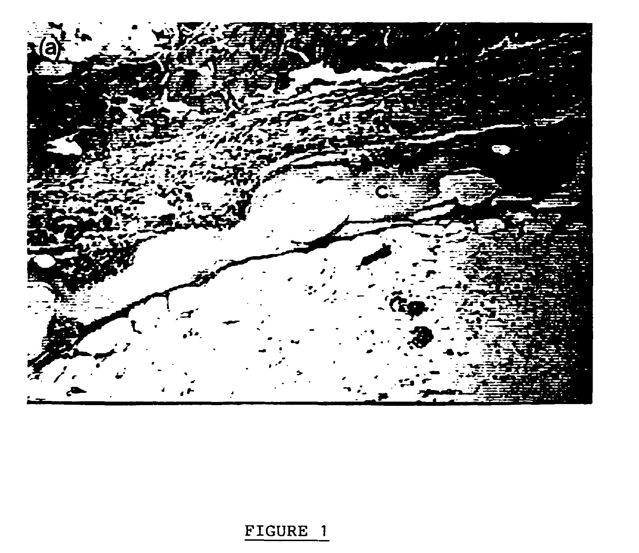

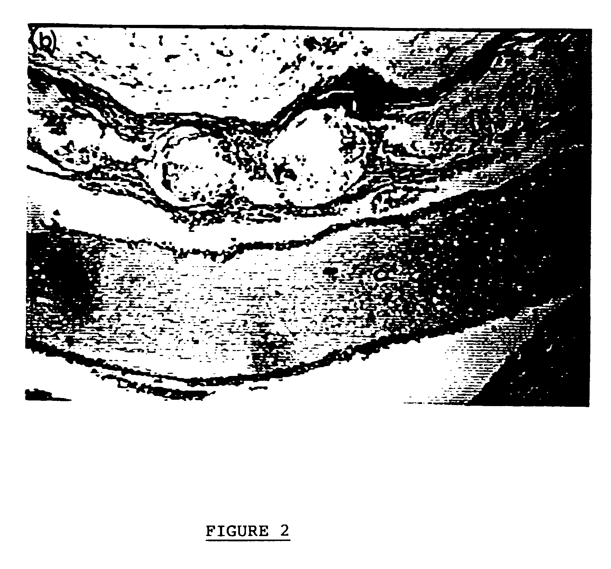

Pieces of film (0.5cm x 1cm) prepared as in Example 1 are implanted subcutaneously in 10wk old Wistar rats, which are sacrificed at 7 and 14 days. The implant and surrounding tissue are excised, fixed, paraffin-wax embedded, sectioned and stained with haematoxylin/eosin. All the films containing oil have retained their integrity and showed significantly less degradation than those without oil at both time-points. This is illustrated in the Figures, in which the remaining reinforced collagen film after 14 days is labelled C. The oil-free film in Figure 1 (comparative example) clearly shows more degradation than the film according to the present invention shown in Figure 2.

## Claims

1. Use of a composite material comprising a collagen matrix reinforced by a layer of a synthetic bioabsorbable material and having oil droplets dispersed in the collagen matrix for the preparation of a bioabsorbable membrane graft in the form of a sheet for use in surgery.

2. Use of a composite material comprising a collagen matrix reinforced by a layer of a synthetic bioabsorbable material and having oil droplets dispersed in the collagen matrix for the preparation of a bioabsorbable graft in the form of a tube for use in surgery.

3. Use of a composite material according to claim 1, 2, wherein the oil droplets comprise from 1% to 75% by weight of the composite surgical material.

4. Use of a composite material according to claim 1, 2 or 3, wherein the oil droplets comprise from 5% to 50% by weight of the surgical material.

5. Use of a composite material according to any preceding claim, wherein the oil droplets comprise from 10% to 40% by weight of the composite surgical material.

6. Use of a composite material according to any preceding claim, wherein the synthetic bioabsorbable material comprises a polymer or copolymer of lactic acid and/or glycolic acid.

7. Use of a composite material according to any preceding claim, wherein the synthetic bioabsorbable material comprises oxidised regenerated cellulose.

8. Use of a composite material according to any preceding claim, wherein the layer of synthetic bioabsorbable material is a knitted, woven or non-woven mesh or web.

9. Use of a composite material according to any preceding claim, wherein said material further comprises an antibiotic, an antiseptic or an anti-inflammatory.

10. Use of a composite material according to any preceding claim, wherein said material further comprises a growth factor, a cytokine or a glycosaminoglycan.

11. Use of a composite surgical material according to any preceding claim which is in the form of a sheet or a tube.

12. Use of a composite surgical material according to any preceding claim for the preparation of a surgical graft or prosthesis.

13. A process to prepare a composite surgical material comprising the steps of:
providing a layer of a synthetic bioabsorbable material;
providing a dispersion of collagen in an oil-in-water emulsion;
coating at least one face of the layer of synthetic bioabsorbable material with the said dispersion; and
drying the composite material thus obtained.

14. A process according to claim 13 wherein the step of providing a dispersion of collagen in an oil-in-water emulsion comprises mixing the collagen and the oil with water followed by emulsifying the oil.

15. A process according to claim 13 or 14, wherein the collagen comprises insoluble fibrous collagen.

16. A process according to claim 13, 14 or 15, wherein the weight ratio of collagen to oil in the said emulsion is from 10:1 to 1:10.

17. A process according to claim 16, wherein the weight ratio of collagen to oil in the said emulsion is from 2:1 to 1:5.

18. A process according to claim 17, wherein the weight ratio of collagen to oil in the said emulsion is from 1:1 to 1:3.

19. A process according to any of claims 13 to 18, wherein the synthetic bioabsorbable material comprises a polymer or copolymer of lactic acid and/or glycolic acid.

20. A process according to any of claims 13 to 19, wherein the synthetic bioabsorbable material comprises oxidised regenerated cellulose.

21. A process according to any of claims 13 to 20, wherein the layer of synthetic bioabsorbable is a knitted, woven or non-woven mesh or web.

22. A process according to any of claims 13 to 21, further comprising the step of dispersing an antibiotic, an antiseptic or an anti-inflammatory compound in the said oil-in-water emulsion.

23. A process according to any of claims 13 to 22, further comprising the step of dispersing a growth factor, a cytokine and/or a glycosamino glycan in the said oil-in-water emulsion.

## Patentansprüche

1. Verwendung eines Verbundmaterials, das eine Kollagenmatrix, die durch eine Schicht eines synthetischen, bio-absorbierbaren Materials verstärkt ist, umfaßt und das in der Kollagenmatrix dispergierte Öltröpfchen aufweist, zur Herstellung eines bio-absorbierbaren Membrantransplantats in der Form eines Blatts zur Verwendung in der Chirurgie.

2. Verwendung eines Verbundmaterials, das eine Kollagenmatrix, die durch eine Schicht eines synthetischen, bio-absorbierbaren Materials verstärkt ist, umfaßt und das in der Kollagenmatrix dispergierte Öltröpfchen aufweist, zur Herstellung eines bio-absorbierbaren Transplantats in der Form einer Röhre zur Verwendung in der Chirurgie.

3. Verwendung eines Verbundmaterials nach Anspruch 1 und 2, wobei die Öltröpfchen 1% bis 75% (Gew.-%) des chirurgischen Verbundmaterials umfassen.

4. Verwendung eines Verbundmaterials nach Ansprüchen 1, 2 oder 3, wobei die Öltröpfchen 5% bis 50% (Gew.-%) des chirurgischen Materials umfassen.

5. Verwendung eines Verbundmaterials nach einem der vorangehenden Ansprüche, wobei die Oltröpfchen 10% bis 40% (Gew.-%) des chirurgischen Verbundmaterials umfassen.

6. Verwendung eines Verbundmaterials nach einem der vorangehenden Ansprüche, wobei das synthetische, bio-absorbierbare Material ein Polymer oder Copolymer von Milchsäure und/oder Glycolsäure umfaßt.

7. Verwendung eines Verbundmaterials nach einem der vorangehenden Ansprüche, wobei das synthetische, bio-absorbierbare Material oxidierte regenerierte Zellulose umfaßt.

8. Verwendung eines Verbundmaterials nach einem der vorangehenden Ansprüche, wobei die Schicht aus synthetischem, bio-absorbierbarem Material ein geknüpftes, gewebtes oder nicht-gewebtes Geflecht oder Netz ist.

9. Verwendung eines Verbundmaterials nach einem der vorangehenden Ansprüche, wobei das Material weiterhin ein Antibiotikum, ein Antiseptikum oder ein Antiphlogistikum umfaßt.

10. Verwendung eines Verbundmaterials nach einem der vorangehenden Ansprüche, wobei das Material weiterhin einen Wachstumsfaktor, ein Zytokin oder ein Glycosaminoglycan umfaßt.

11. Verwendung eines chirurgischen Verbundmaterials nach einem der vorangehenden Ansprüche, das in Form eines Blatts oder einer Röhre vorliegt.

12. Verwendung eines chirurgischen Verbundmaterials nach einem der vorangehenden Ansprüche zur Herstellung eines chirurgischen Transplantats oder einer Prothese.

13. Verfahren zur Herstellung eines chirurgischen Verbundmaterials, das die folgenden Schritte umfaßt:
Bereitstellen einer Schicht eines synthetischen, bio-absorbierbaren Materials;
Bereitstellen einer Dispersion aus Kollagen in einer Öl-in-Wasser-Emulsion;
Beschichten mindestens einer Fläche der Schicht aus synthetischem, bio-absorbierbarem Material mit der Dispersion; und
Trocknen des dadurch erhaltenen Verbundmaterials.

14. Verfahren nach Anspruch 13, wobei der Schritt des Bereitstellens einer Dispersion aus Kollagen in einer Öl-in-Wasser-Emulsion das Mischen des Kollagens und des Öls mit Wasser, gefolgt vom Emulgieren des Öls umfaßt.

15. Verfahren nach Anspruch 13 oder 14, wobei das Kollagen unlösliches, fibrilläres Kollagen umfaßt.

16. Verfahren nach Anspruch 13,14 oder 15, wobei das Gewichtsverhältnis von Kollagen zu Öl in der Emulsion 10:1 bis 1:10 beträgt.

17. Verfahren nach Anspruch 16, wobei das Gewichtsverhältnis von Kollagen zu Öl in der Emulsion 2:1 bis 1:5 beträgt.

18. Verfahren nach Anspruch 17, wobei das Gewichtsverhältnis von Kollagen zu Öl in der Emulsion 1:1 bis 1:3 beträgt.

19. Verfahren nach einem der Ansprüche 13 bis 18, wobei das synthetische, bio-absorbierbare Material ein Polymer oder Copolymer von Milchsäure und/oder Glycolsäure umfaßt.

20. Verfahren nach einem der Ansprüche 13 bis 19, wobei das bio-absorbierbare, synthetische Material oxidierte Regeneratzellulose umfaßt.

21. Verfahren nach einem der Ansprüche 13 bis 20, wobei die Schicht aus bio-absorbierbarem, synthetischem Material ein geknüpftes, gewebtes oder nicht-gewebtes Geflecht oder Netz ist

22. Verfahren nach einem der Ansprüche 13 bis 21, das weiterhin den Schritt des Dispergierens einer antibiotischen, einer antiseptischen oder einer antiphlogistischen Verbindung in der Öl-in-Wasser-Emulsion umfaßt.

23. Verfahren nach einem der Ansprüche 13 bis 22, das weiterhin den Schritt des Dispergierens eines Wachstumsfaktors, eines Zytokins und/oder eines Glycosaminoglycans in der Öl-in-Wasser-Emulsion umfaßt.

## Revendications

1. Utilisation d'un matériau composite comprenant une matrice de collagène renforcée par une couche d'un matériau bioabsorbable synthétique et ayant des gouttelettes d'huile dispersées dans la matrice de collagène pour la préparation d'un greffon membranaire bioabsorbable sous la forme d'une feuille pour utilisation en chirurgie.

2. Utilisation d'un matériau composite comprenant une matrice de collagène renforcée par une couche d'un matériau bioabsorbable synthétique et ayant des gouttelettes d'huile dispersées dans la matrice de collagène pour la préparation d'un greffon bioabsorbable sous la forme d'un tube pour utilisation en chirurgie.

3. Utilisation d'un matériau composite selon la revendication 1, 2, dans laquelle les gouttelettes d'huile comprennent de 1 % à 75 % en poids du matériau chirurgical composite.

4. Utilisation d'un matériau composite selon la revendication 1, 2 ou 3, dans laquelle les gouttelettes d'huile comprennent de 5 % à 50 % en poids du matériau chirurgical.

5. Utilisation d'un matériau composite selon l'une quelconque des revendications précédentes, dans laquelle les gouttelettes d'huile comprennent de 10 % à 40 % en poids du matériau chirurgical composite.

6. Utilisation d'un matériau composite selon l'une quelconque des revendications précédentes, dans laquelle le matériau bioabsorbable synthétique comprend un polymère, ou un copolymère, d'acide lactique et/ou d'acide glycolique.

7. Utilisation d'un matériau composite selon l'une quelconque des revendications précédentes, dans laquelle le matériau bioabsorbable synthétique comprend une cellulose régénérée oxydée.

8. Utilisation d'un matériau composite selon l'une quelconque des revendications précédentes, dans laquelle la couche de matériau bioabsorbable synthétique est une nappe ou une bande tricotée, tissée ou non-tissée.

9. Utilisation d'un matériau composite selon l'une quelconque des revendications précédentes, dans laquelle ledit matériau comprend, en outre, un antibiotique, un antiseptique ou un anti-inflammatoire.

10. Utilisation d'un matériau composite selon l'une quelconque des revendications précédentes, dans laquelle ledit matériau comprend, en outre, un facteur de croissance, une cytokine ou un glycosaminoglycane.

11. Utilisation d'un matériau chirurgical composite selon l'une quelconque des revendications précédentes qui se présente sous la forme d'une feuille ou d'un tube.

12. Utilisation d'un matériau chirurgical composite selon l'une quelconque des revendications précédentes pour la préparation d'un greffon chirurgical ou d'une prothèse.

13. Procédé de préparation d'un matériau chirurgical composite comprenant les étapes consistant à :
former une couche d'un matériau bioabsorbable synthétique ;
former une dispersion de collagène dans une émulsion huile-dans-l'eau ;
revêtir au moins une face de la couche du matériau bioabsorbable synthétique de ladite dispersion ; et
sécher le matériau composite ainsi obtenu.

14. Procédé selon la revendication 13, dans lequel l'étape consistant à former une dispersion de collagène dans une émulsion huile-dans-l'eau comprend le mélange du collagène et de l'huile avec de l'eau suivie par l'émulsification de l'huile.

15. Procédé selon la revendication 13 ou 14, dans lequel le collagène comprend un collagène fibreux insoluble.

16. Procédé selon la revendication 13, 14 ou 15, dans lequel le rapport en poids du collagène à l'huile dans ladite émulsion est de 10:1 à 1:10.

17. Procédé selon la revendication 16, dans lequel le rapport en poids du collagène à l'huile dans ladite émulsion est de 2:1 à 1:5.

18. Procédé selon la revendication 17, dans lequel le rapport en poids du collagène à l'huile dans ladite émulsion est de 1:1 à 1:3.

19. Procédé selon l'une quelconque des revendications 13 à 18, dans lequel le matériau bioabsorbable synthétique comprend un polymère, ou un copolymère, d'acide lactique et/ou d'acide glycolique.

20. Procédé selon l'une quelconque des revendications 13 à 19, dans lequel le matériau bioabsorbable synthétique comprend une cellulose régénérée oxydée.

21. Procédé selon l'une quelconque des revendications 13 à 20, dans lequel la couche de bioabsorbable synthétique est une nappe ou une bande tricotée, tissée ou non-tissée.

22. Procédé selon l'une quelconque des revendications 13 à 21, comprenant en outre l'étape consistant à disperser un antibiotique, un antiseptique ou un composé anti-inflammatoire dans ladite émulsion huile-dans-l'eau.

23. Procédé selon l'une quelconque des revendications 13 à 22, comprenant en outre l'étape consistant à disperser un facteur de croissance, une cytokine et/ou un glycosamino glycane dans ladite émulsion huile-dans-l'eau.
